# EUROPÄISCHE PATENTANMELDUNG

(11) **EP 2 457 596 A1**
(43) Veröffentlichungstag der Anmeldung: **30.05.2012**
(21) Anmeldenummer: 11009140.2
(22) Anmeldetag: 17.11.2011
(51) Int. Cl.: A61L 2/232, A01N 25/24, A01N 25/34, E05B 1/00

(54) **Antimikrobielle Oberflächenschicht mit eingelagerten Aktivkohlekugeln**

(30) Priorität: 29.11.2010 DE 202010015893 U; 21.04.2011 DE 202011005569 U
(71) Anmelder: Kahrom, Kami Klaus, 64390 Erzhausen (DE)
(72) Erfinder: Kahrom, Kami Klaus, 64390 Erzhausen (DE)
(74) Vertreter: Gallo, Wolfgang

(57) **Zusammenfassung**

Antimikrobiell wirkende Oberflächenschicht eines Substratkörpers wie beispielsweise eines Türgriffs, die aus einem antimikrobiell wirkenden Material oder einem ein antimikrobiell wirkendes Material enthaltenden Werkstoff besteht, wobei in die Oberfläche der antimikrobiellen Oberflächenschicht sphärische Adsorbenzien in Gestalt von Aktivkohle-Kügelchen eingelagert sind.

## Beschreibung

Die Erfindung betrifft eine antimikrobielle Oberflächenschicht mit eingelagerten Adsorbenzien, die beispielsweise zur Beschichtung von Türgriffen Anwendung finden kann. Diese Oberflächenschicht kann durch eine auf einen Substratkörper aufgebrachte Beschichtung oder durch die oberste Materialschicht des Substratkörpers selbst gebildet sein.

Bekanntermaßen können Oberflächen, die gewöhnlich von Menschen berührt werden, wie insbesondere Türgriffe, Lichtschalter, Drücker für Toilettenspülung usw., als Überträger von Bakterien, Viren und Pilzen fungieren. Wenn also beispielsweise eine vollgeschnupfte Hand eine Türklinke berührt, bleiben dort Viren haften und werden auf die nächste Hand, die die Türklinke berührt, übertragen.

Antimikrobielle Oberflächen, entweder in Gestalt von antimikrobiellen Oberflächenbeschichtungen auf Gegenständen oder in Gestalt der Oberflächen von aus antimikrobiell wirkenden Materialien gefertigten Gegenständen, sind bekannt. Ihre Besonderheit besteht darin, dass sie aus Edelmetallen bestehen oder aus Kunststoffen, die entsprechende Additive enthalten, die Edelmetallionen mit antimikrobieller Wirkung freisetzen. Solche antimikrobiellen Oberflächen können beispielsweise durch Pulverbeschichtungen aus entsprechenden Materialien hergestellt sein, oder können Oberflächen von Gegenständen aus Kupferlegierungen oder Kunststoffen sein, wie sie beispielsweise unter der Marke RO-WAcare erhältlich sind, die ein antimikrobielles Additiv als Wirkstoff beinhalten. Solche Materialien haben die Eigenschaft, innerhalb weniger Stunden Bakterien, Viren und Pilze zu eliminieren.

Bei stark frequentierten Oberflächen, wie beispielsweise bei Türgriffen in oder an öffentlichen Gebäuden oder von Toilettentüren, ist ein Zeitraum von selbst wenigen Stunden für eine Mikroben abtötende Wirkung zu viel, um eine Wirkung herbei zu führen. Denn bevor die antimikrobielle Wirkung einsetzen kann, findet eine erneute Verkeimung durch die nächste Berührung statt.

Aufgabe der Erfindung ist es daher, eine antimikrobielle Oberflächenbeschichtung für kritische Oberflächen der genannten Kategorie zu schaffen, die eine schnell wirkende antimikrobielle Wirkung entfaltet, um eine wirkungsvolle Unterbindung einer möglichen Mikrobenübertragung bei relativ häufig aufeinander folgenden Berührungen erreichen zu können.

Diese Aufgabe wird gemäß der Erfindung durch die im Anspruch 1 angegebene antimikrobielle Beschichtung gelöst. Vorteilhafte Ausgestaltungen der Erfindung sind Gegenstand der Unteransprüche.

Die Erfindung beruht auf der Überlegung, dass es zum Erreichen einer Verhinderung einer Mikrobenübertragung darauf ankommt, für ein Festhalten von auf eine berührte Fläche übertragenen Mikroben zu sorgen, damit diese bei erneuter Berührung sich nicht wieder von der Oberfläche lösen können, sondern dort verhaftet bleiben, bis die abtötende Wirkung des antimikrobiellen Materials eingetreten ist.

Dementsprechend sieht die Erfindung die Einlagerung sphärischer Adsorbenzien in Gestalt von Aktivkohle-Kügelchen in die antimikrobielle Beschichtung vor. Diese sphärischen Adsorbenzien haben die Wirkung, dass sie auf die Oberfläche aufgebrachte Keime adsorbieren, die dadurch festgehalten werden, bis sie durch austretende Metallionen des antimikrobiellen Materials eliminiert werden.

Sphärische Adsorbenzien sind an sich bereits bekannt. Sie finden ihren größten Einsatz im Militärbereich, wo sie zur Ausrüstung von Bekleidungsstücken eingesetzt werden, damit biologische oder chemische Kampfstoffe in den Adsorbenzien aufgenommen werden und nicht durch die Kleidung der Soldaten hindurch dringen können.

Die Erfindung kombiniert also in vorteilhafter Weise die antimikrobielle Wirkung entsprechender Materialien mit den adsorbierenden Eigenschaften von sphärischen Adsorbenzien.

Die Erfindung wird nachstehend beispielshalber mehr im einzelnen beschrieben.

Die gemäß der Erfindung zu verwendenden sphärischen Adsorbenzien können aus künstlichen Harzkügelchen gewonnen werden und sind durch Wahl der Verfahrensparameter exakt reproduzierbar. Die Harzkügelchen werden in Drehöfen auf ca. 1.700 °C erhitzt. Übrig bleibt dann ein offenporiger Kohlenstoffkörper in Kugelform, der abriebfest und druckbeständig ist. Es handelt sich dabei um Aktivkohle. Durch entsprechende Wahl der eingesetzten Chemikalien und Temperaturkurven kann die Ausbildung der Poren beeinflusst werden. Für den erfindungsgemäßen Zweck werden die Poren so angelegt, dass auf die Oberfläche gelangende Keime möglichst schnell von der Oberfläche nach innen transportiert werden, wo sie der Wirkung des antimikrobiellen Materials ausgesetzt werden können. Diese Aktivkohle-Kügelchen haben eine enorm große innere Oberfläche. Rein mathematisch betrachtet, hat eine Masse von einem Gramm solcher Aktivkohle-Kügelchen eine innere Oberfläche, die etwa der Größe der Fläche von mehr als fünf Tennisplätzen entspricht.

Zur Herstellung einer antimikrobiellen Beschichtung mit eingelagerten sphärischen Adsorbenzien nach der Erfindung werden Substratkörper, wie beispielsweise Türklinken, aus temperaturbeständigen Materialien wie Metall oder Glas mit antimikrobiell ausgestattetem Beschichtungspulver auf der Oberfläche benetzt und erwärmt, bis das Beschichtungspulver einen gelartigen Zustand einnimmt. In diesem gelierten Zustand des Beschichtungspulvers werden dann die sphärischen Adsorbenzien auf die Oberfläche aufgebracht. Das Aufbringen der sphärischen Adsorbenzien soll möglichst gleichmäßig auf der Oberfläche verteilt erfolgen, und die Aktivkohle-Kügelchen sollen etwa zur Hälfte in der gelierten Pulvermasse verankert werden. Da die Adsorbenzien sehr temperaturbeständig sind, können auch antimikrobielles Kupfer oder andere Edelmetalle in viskosem Zustand mit den Adsorbenzien beaufschlagt werden, um die gleiche Wirkung zu erzielen. Wenn also beispielsweise der Körper eines Türgriffs aus antimikrobiellem Kupfer oder einer Kupferlegierung oder aus einem anderen Edelmetall besteht, kann der Oberflächenbereich dieses Substratkörpers bis in einen viskosen Zustand erhitzt werden und dann mit den Adsorbenzien beaufschlagt werden, um die gleiche Wirkung zu erzielen.

Für den Fall, daß der Substratkörper nicht aus einem temperaturbeständigen Material besteht, ist es aber auch möglich, das antimikrobielle Aditiv einschließlich der sphärischen

Adsorbenzien mittels geeigneter Klebstoffe auf den Substratkörper aufzubringen. Zwar ist in diesem Fall die Fixierung auf dem Substratkörper nicht so gut und nicht so dauerhaft wie bei der oben beschriebenen Einbettung in hitzebeständige Substratmaterialien, aber es funktioniert hinreichend gut und erlaubt dadurch eine ganz erhebliche Ausweitung des Anwendungsbereichs, da damit auch Materialien wie nicht hitzebeständige Kunststoffe, Pappe bzw. Karton, oder Glas antimikrobiell beschichtbar sind.

Dadurch wird eine Oberflächenschicht erzielt, die eine antimikrobielle Wirkung mit einer Adsorptionswirkung kombiniert und daher aufgebrachte Keime festhält, so dass sie nicht mehr weiter abgegeben werden können, bis sie durch die abtötende antimikrobielle Wirkung des antimikrobiellen Materials eliminiert worden sind.

Die erfindungsgemäße antimikrobielle Beschichtung mit eingelagerten sphärischen Adsorbenzien kann also, wie oben dargestellt, entweder durch eine auf einem Substratkörper aufgebrachte Beschichtung aus einem Beschichtungsmaterial mit Einlagerung der sphärischen Adsorbenzien bestehen, oder sie kann aus der durch Erhitzung und Einlagerung der sphärischen Adsorbenzien modifizierten Oberflächenschicht eines aus einem antimikrobiellen Material bestehenden Substratkörpers gebildet sein.

## Patentansprüche

1. Antimikrobiell wirkende Oberflächenschicht eines Substratkörpers wie beispielsweise eines Türgriffs, die aus einem antimikrobiell wirkenden Material oder einem ein antimikrobiell wirkendes Material enthaltenden Werkstoff besteht, **dadurch gekennzeichnet, dass** in die Oberfläche der antimikrobiellen Oberflächenschicht sphärische Adsorbenzien in Gestalt von Aktivkohle-Kügelchen eingelagert sind.

2. Antimikrobielle Oberflächenschicht nach Anspruch 1, die durch eine auf einem Substratkörper aufgebrachte Beschichtung aus antimikrobiellem oder einem ein antimikrobielles Material enthaltenden Beschichtungsmaterial gebildet ist, in dessen Oberfläche die sphärischen Adsorbenzien eingelagert sind.

3. Antimikrobielle Oberflächenschicht nach Anspruch 1, die durch die oberste Materialschicht eines aus einem antimikrobiell wirkenden Werkstoff bestehenden oder aus einem ein antimikrobiell wirkendes Material enthaltenden Werkstoff bestehenden Substratkörpers ist, in dessen Oberfläche die sphärischen Adsorbenzien eingelagert sind.

4. Antimikrobielle Oberflächenschicht nach Anspruch 1, die mittels eines Klebstoffs gebundenes und auf dem Substratkörper fixiertes antimikrobiell wirkendes Material mit eingelagerten sphärischen Adsorbenzien gebildet ist.

5. Verfahren zur Herstellung einer antimikrobiellen Oberflächenschicht nach Anspruch 2, bei welchem ein aus einem antimikrobiell wirkenden Werkstoff bestehendes oder ein antimikrobiell wirkendes Material enthaltenden Werkstoff bestehendes Beschichtungsmaterial auf der Oberfläche eines Substratkörpers aufgebracht und bis zum Erreichen eines gelartigen Zustands erhitzt wird, worauf die sphärischen Adsorbenzien auf die Oberfläche des in gelierten Zustand befindlichen Beschichtungsmaterials aufgebracht und in diese eingelagert werden.

6. Verfahren zur Herstellung einer antimikrobiellen Oberflächenschicht nach Anspruch 3, wobei die oberste Materialschicht eines aus einem antimikrobiell wirkenden Werkstoff bestehenden oder ein antimikrobiell wirkendes Material enthaltenden Werkstoff bestehenden Substratkörpers erhitzt wird, bis die Oberflächenschicht einen gelartigen Zustand oder Erweichungszustand erreicht, worauf die sphärischen Adsorbenzien auf die Oberfläche und in diese eingelagert werden.

7. Verfahren zur Herstellung einer antimikrobiellen Oberflächenschicht nach Anspruch 4, wobei antimikrobiell wirkendes Material sowie sphärische Adsorbenzien mittels eines Klebstoffs gebunden und auf der Oberfläche des Substratkörpers fixiert werden.
